# EUROPEAN PATENT APPLICATION

(11) **EP 1 043 301 A2**
(43) Date of publication of application: **11.10.2000**
(21) Application number: 00302357.9
(22) Date of filing: 22.03.2000
(51) Int. Cl.: C07C 51/12, C07C 53/08, C07C 53/12, C07C 67/36, C07C 69/14

(54) **Carbonylation process**

(30) Priority: 06.04.1999 GB 9907836
(71) Applicant: BP Chemicals Limited, London EC2M 7BA (GB)
(72) Inventor: Ditzel, Evert Jan, Hull, HU12 8DS (GB); Poole, Andrew David, Hull, HU12 8DS (GB); Cole-Hamilton, David John, Fife, KY16 9ST, Scotland (GB); Marr, Andrew Craig, Nottingham, N47 1LP (GB)
(74) Representative: Perkins, Nicholas David

(57) **Abstract**

A process for the carbonylation of an alcohol and/or reactive derivative thereof which process comprises contacting a liquid reaction composition comprising an alcohol and/or a reactive derivative thereof, a hydrocarbyl halide co-catalyst and optionally water, optionally with hydrogen at a molar ratio of hydrogen : carbon monoxide of less than 1 : 10, in the presence of a rhodium catalyst having the following structural formula (I) : 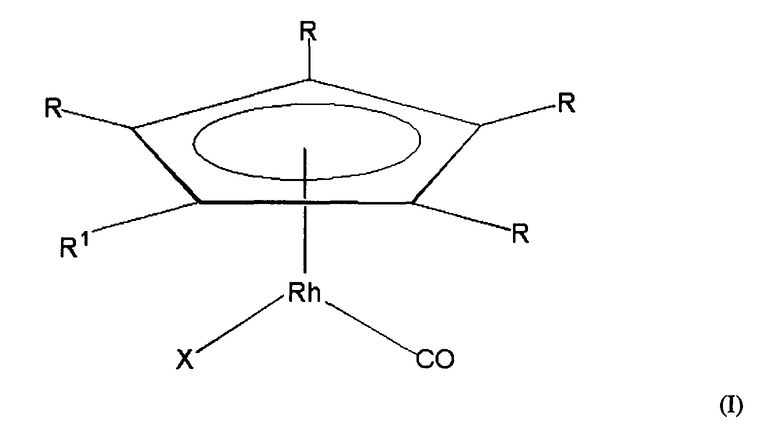 wherein :
the R groups are independently hydrogen, alkyl, aryl, substituted alkyl or aryl group; the R groups may be the same or different to each other;
R¹ is hydrogen, alkyl, aryl, substituted alkyl or aryl or a bridging group to X; and
X is a two electron donor which may optionally be linked to the cyclopentadienyl group via R¹
and the Rh may additionally be bound to a Z group such that Z is halide, in which case in place of the CO group there may be an acyl group of formula R⁴CO in which R⁴ is hydrogen, alkyl, aryl or substituted alkyl or aryl.

## Description

The present invention relates to a process for the carbonylation of an alcohol and/or a reactive derivative thereof and in particular to a process carded out in the presence of a rhodium catalyst.

Acetic acid and acetic anhydride have been known as industrial chemicals for many years. Acetic anhydride constitutes the second largest end-use for acetic acid and is widely employed in the production of cellulose acetate, and other cellulose esters. Acetic acid is used as a preservative, as an intermediate in the production of acetate esters and as a co-reactant in the production of vinyl acetate.

The production of acetic acid by the liquid phase carbonylation of methanol is a well known industrially operated process and is widely operated commercially. The carbonylation process is typically catalysed by rhodium and methyl iodide and is described in Howard et al in *Catalysis Today* 18 (1993) 325-354 as well as in US 3769329 and GB 1233121. EP 0087870 describes a process for the production of acetic anhydride in the presence of a rhodium catalyst.

EP-A- 0029723 describes the use of cyclopentadienyl complexes of Group VIII metal other than iron, palladium and platinum in homologation reactions of methanol with synthesis gas ( a mixture of hydrogen and carbon monoxide) to produce ethanol and/or acetaldehyde. According to EP-A-0029723 the molar ratio of carbon monoxide: hydrogen is suitably in the range 2:1 to 1:3. The present invention is a carbonylation process which does not use significant amounts of hydrogen, if any.

We have now found that carboxylic acid and/or carboxylic acid anhydride can be produced using a rhodium cyclopentadienyl catalyst in a carbonylation process even at low concentrations of hydrogen or in the substantial absence of hydrogen.

Accordingly, the present invention provides a process for the carbonylation of an alcohol and/or a reactive derivative thereof which process comprises contacting a liquid reaction composition comprising an alcohol and/or a reactive derivative thereof, a hydrocarbyl halide co-catalyst and optionally water, optionally with hydrogen at a molar ratio of hydrogen : carbon monoxide of less than 1 : 10, in the presence of a rhodium catalyst having the following structural formula (I): 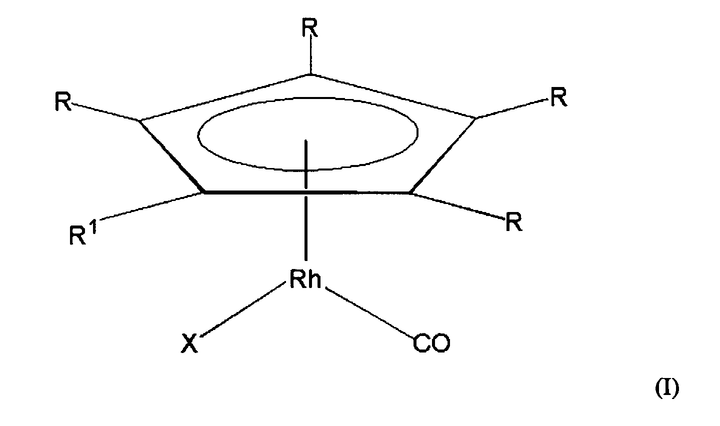 wherein :
the R groups are independently hydrogen, alkyl, aryl, substituted alkyl or aryl group; the R groups may be the same or different to each other;
R¹ is hydrogen, alkyl, aryl, substituted alkyl or aryl or a bridging group to X; and
X is a two electron donor which may optionally be linked to the cyclopentadienyl group via R¹.

We have now found that the aforementioned catalyst system can be used in the production of carboxylic acids and carboxylic acid anhydrides in particular when the process is carried out with only minor amounts of hydrogen or in the absence of hydrogen.

In the carbonylation process of the present invention, the alcohol may suitably be an aliphatic alcohol having up to 6 carbon atoms, for example methanol, ethanol, propanols, butanols, pentanols and hexanols. The carbonylation product of an alcohol having n carbon atoms will comprise a carboxylic acid having n + 1 carbon atoms. If the alcohol is present in excess in the reaction composition the product will comprise carboxylic acid and/or the ester of the carboxylic acid and the alcohol reactant. In the absence of water and under anhydrous conditions the carbonylation product will comprise the carboxylic acid and/or its anhydride. A preferred alcohol is methanol, the carbonylation product of which comprises acetic acid and/or methyl acetate, and/or acetic anhydride under anhydrous conditions.

Alternatively or additionally, reactive derivatives of the alcohol may be used, for example alkyl halides, dialkyl ethers and esters of alcohols having n carbon atoms with the carboxylic acid product having n+1 carbon atoms. Suitable reactive derivatives of methanol include, for example, methyl acetate, dimethyl ether and methyl iodide. A mixture of more than one alcohol and/or reactive derivative may be used, for example methanol and methyl acetate.

In the carbonylation process of the present invention, the carboxylic acid ester reactant may suitably be a C₁ to C₆ carboxylic acid and a C₁ to C₆ monofunctional aliphatic alcohol. Preferably, the ester reactant is an ester of a carboxylic acid and methanol, ethanol or propanol. Particularly preferred, is methyl acetate. A mixture of esters may be employed.

The reactive derivative may be a C₁ to C₆ hydrocarbyl ether, preferably a dialkyl ether, most preferably dimethyl ether, diethyl ether or dipropyl ether. A mixture of ethers may be used.

A preferred reactant is methyl acetate and/or dimethyl ether optionally with methanol and/or water to produce acetic anhydride and optionally acetic acid.

When water is present in the reaction system, the carbonylation process of the present invention produces carboxylic acid. Suitably when present, water is present in an amount of greater than 0.1% up to 25% by weight based on the weight of reaction composition. If the reactant is an alcohol, water is generally produced during the carbonylation process as a by-product of esterification. The water produced, may be recycled to the reaction composition.

When the carbonylation process of the present invention produces carboxylic acid anhydride, the process is carried out in the absence of water.

The presence of hydrogen in the carbon monoxide feed and generated in situ by the water gas shift reaction is preferably kept low, for example, less than 2 bar partial pressure, as its presence may result in the formation of hydrogenation products. Whilst increasing the partial pressure of hydrogen in a carbonylation process performed under substantially anhydrous conditions may increase the rate of reaction, a disadvantage is that it also increases the formation of by-products. An advantage of the present invention is that the rate of reaction can be maintained at low partial pressure of hydrogen. This has the advantage of reducing by-product formation whilst maintaining rate of reaction. Thus, for example, the hydrogen partial pressure may be in the range up to 1 bar. The molar ratio of hydrogen (if present) : carbon monoxide is less than 1:10 in the reactor. Preferably, the carbonylation process is carried out substantially in the absence of hydrogen.

The carbonylation process is carried out in the presence of a hydrocarbyl halide co-catalyst. The hydrocarbyl halide co-catalyst may be a C₁ to C₆ halide for example methyl, ethyl or propyl halide. The halide may be any halide, preferably iodide. The preferred hydrocarbyl halide is methyl iodide. The concentration of hydrocarbyl halide co-catalyst in the reaction composition is suitably in the range of from 1 to 30% by weight, preferably 1 to 20% by weight, most preferably 5 to 20% by weight.

The carbonylation process may be carded out in the presence of a promoter. Suitable promoters may be selected from Group IA and Group IIA metal iodides, quaternary ammonium iodides and phosphonium iodides, optionally prepared in-situ. The concentration of promoter in the reaction composition is suitably equivalent to up to 20% by weight iodide, preferably up to 15% by weight iodide.

Alternatively, the promoter may comprise ruthenium, osmium, rhenium or manganese. The promoter is suitably present in the liquid reaction composition at a molar ratio of promoter : rhodium of 0.2 : 1 to 20 : 1.

Carbon monoxide, used in the process of the present invention, may be essentially pure or may contain inert impurities such as carbon dioxide, methane, nitrogen, noble gases and C₁ to C₄ paraffinic hydrocarbons.

The carbonylation process is carried out in the presence of a rhodium-cyclopentadienyl compound.

The R groups of formula (I) may be independently selected from hydrogen, alkyl, aryl or substituted alkyl or aryl. Each R may be the same or may be different. Preferably R is alkyl, preferably methyl.

The cyclopentadienyl group may be part of a larger ring system, for example indenyl or flourenyl.

R¹ of formula (I) may be hydrogen, alkyl, aryl or substituted alkyl or aryl group, preferably methyl. R¹ may be a bridging group to X. For example, where R¹ is a bridging group, R¹ may be (CR² ₂)ₙYₘ where n is an integer 0 to 5 and R² groups are independently hydrogen, alkyl, aryl, substituted alkyl or aryl, Y is a group containing silicon, oxygen, nitrogen or sulphur and m is an integer 0 to 5. The R² groups may be the same or different to each other.

X of formula (I) may be a two electron donor, for example CO, PR³₃, NR³₃, SR³₂ OR³₂ where each R³ is independently hydrogen, alkyl, aryl or substituted alkyl or aryl which may be the same or different. When R¹ is a bridging group, suitable two electron donors include PR''₂, NR''₂, SR'', OR'' where R'' is alkyl, aryl or substituted alkyl or aryl which may be the same or different. The P, S, N and O atoms may also be part of a ring system, for example an aromatic system such as pyridine. X may also be a charged species, for example a halide, preferably iodide, in which case the catalyst precursor is also charged.

In a further embodiment of the present invention, the Rh of formula (I) is additionally bound to a Z group, such that Z = X = halide, preferably iodide. The rhodium catalyst thus has the structural formula (II) : 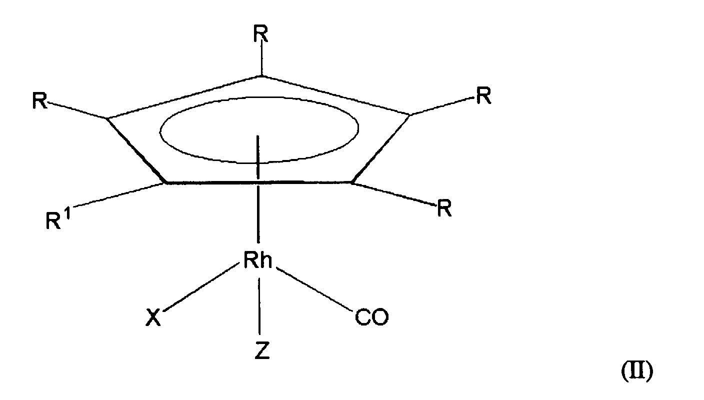 wherein R and R¹ are as hereinbefore defined for formula (I) and X and Z are halide, preferably iodide.

In yet a further embodiment of the present invention, the Rh of formula (I) is additionally bound to a Z group, such that Z is halide, preferably iodide and in place of the CO group of formula (I) there is an acyl group of formula R⁴CO in which R⁴ is hydrogen, alkyl, aryl or substituted alkyl or aryl. Preferably R⁴ is alkyl, preferably methyl. The rhodium catalyst thus has the structural formula (III) : 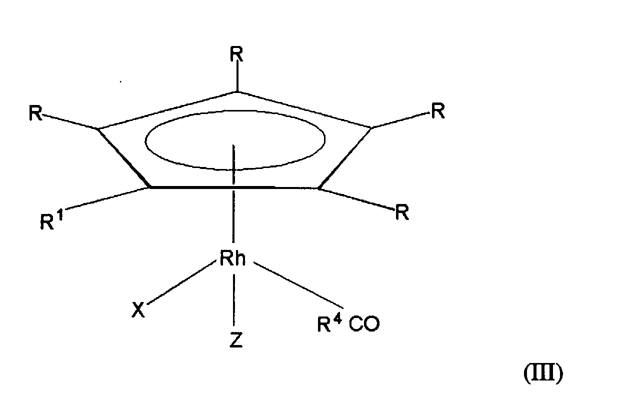 wherein R, R¹ and X are as hereinbefore defined for formulae (I) and (II), Z is halide, preferably iodide and R⁴ is hydrogen, alkyl, aryl or substituted alkyl or aryl. Preferably R⁴ is alkyl, preferably methyl.

Suitably, the rhodium catalyst is Cp*Rh(CO)₂ or Cp*Rh(CO)I₂ in which Cp* is pentamethyl cyclopentadienyl group.

The catalyst precursor may be prepared prior to addition to the liquid reaction composition or may be prepared *in situ* in the liquid reaction composition. A suitable method of preparing the catalyst precursor *in situ* is to react in the liquid reaction composition, a rhodium cyclopentadienyl compound of formula (I), (II) or (III) in which X is CO with a reagent capable of substituting the CO with a group having the formula X other than CO. Suitable reagents include PR³₃, NR³₃, SR³₂ and OR³₂.

The concentration of the rhodium-cyclopentadienyl compound in the liquid reaction composition is preferably in the range of from 10 to 1500 ppm by weight of rhodium.

The carbonylation process may be carried out under a pressure in the range of from 1 to 100 barg, preferably 20 to 50 barg and a temperature suitably in the range of 130 to 250°C, preferably in the range of 170 to 200°C.

The carbonylation process may be carried out as a batch or a continuous process. Preferably, the process is a continuous process.

The carbonylation product comprising carboxylic acid and/or carboxylic acid anhydride and/or ester product may be removed from the reactor by withdrawing the liquid reaction composition and separating the product from the other components in the liquid mixture by one or more flash and/or distillation stages. The product may be removed as a vapour from the reactor.

The invention will now be illustrated by way of the following examples in which Me represents CH₃.

### Example 1 (Formula (I) R and R¹ are Me and X is CO) - Production of Acetic Acid

A 300 ml Hastelloy B2 autoclave equipped with a dispersimax stirrer, a ballast vessel and a liquid catalyst injection system was used for a series of batch carbonylation experiments. A gas supply to the autoclave was provided from a gas ballast vessel, feed gas being provided to maintain the autoclave at a constant pressure and the rate of gas uptake being calculated (with an accuracy believed to be ± 1%) from the rate at which the pressure falls in the ballast vessel.

At the end of the experiment liquid samples from the autoclave were analysed by gas chromatography.

The rhodium complex, methyl acetate, acetic acid, water were all weighed out into the autoclave under nitrogen inside a glove bag, and the autoclave sealed. The autoclave was then removed and the unit assembled. The autoclave headspace was flushed once with carbon monoxide and then pressurised to ca. 8 barg with carbon monoxide at room temperature. The autoclave was heated with stirring (1500 ppm) to 185°C. Once at temperature methyl iodide was injected into the autoclave from the catalyst injection facility using an overpressure of carbon monoxide. The overpressure was calculated so that after injection the desired reaction pressure of 28 barg was attained. After catalyst injection the autoclave pressure was kept constant (± 0.5 barg) using carbon monoxide fed from the ballast vessel.

On completion the autoclave was isolated from the gas supply and the reactor contents cooled to room temperature. The autoclave was vented and the vent gases sampled and analysed. The liquid reaction composition was discharged from the autoclave, sampled and was analysed for liquid products. Acetic acid production was calculated on the basis of carbon monoxide uptake and consumption.

### Example 2 (Formula (I) R and R¹ are Me and X is CO) - Production of Acetic Acid

The procedure of Example 1 was repeated wherein a 30 ml Hastelloy B2 autoclave equipped with a mechanical stirrer, a ballast vessel and a liquid catalyst injection system was used. A gas supply to the autoclave was provided from a gas ballast vessel, feed gas being provided to maintain the autoclave at a constant pressure and the rate of gas uptake being calculated from the rate at which the pressure falls in the ballast vessel.

A stock solution was prepared containing acetic acid (64.0g), methyl acetate (15.0g) and water (7.0g). The stock solution was degassed by bubbling argon through it. [(C₅Me₅)Rh(CO)₂] was dissolved in the stock solution (4.0g) under argon. The apparatus was flushed with argon and an aliquot of the resulting solution injected into the autoclave. The autoclave was pressurised to 10 barg with carbon monoxide and stirred. The autoclave was heated with stirring to 185°C. The methyl iodide in stock solution was then injected into the autoclave using an overpressure of carbon monoxide. The overpressure was calculated so that on injection the desired reaction pressure was achieved. After catalyst injection the autoclave pressure was kept constant using carbon monoxide fed from the ballast vessel.

On completion the autoclave was isolated from the gas supply and the reactor contents cooled to room temperature. The autoclave was vented degassed and the liquid reaction composition was discharged from the autoclave.

### Production of Acetic Anhydride

### Example 3 :formula (I) R and R¹ are Me and X is CO

### Example 4 : formula (II) X=Z=I, R and R¹ are Me.

The procedure of Example 2 was repeated wherein a stock solution was prepared containing acetic acid (96.70g), methyl acetate (47.70g), acetic anhydride (1.00g). This was degassed by bubbling argon through it.

The Rh complex and LiI were weighed into a Schlenk tube along with 4.4 cm⁻³ of the stock solution. This was then injected into the autoclave and the reaction carried out in the same manner as in Example 2 above.

### Comparative Experiment A

The procedure of Examples 3 and 4 was followed using RhCl₃.3H₂O as the catalyst precursor. This forms [RhI₂(CO)₂]⁻ in situ so there was an induction period, but the reaction rate was determined from the linear portion of the graph of carbon monoxide up-take versus time.

The autoclave charges relating to each example and comparative experiment are given in Tables 1 and 2 together with the reaction rates and turnover numbers. In the Tables Cp* represents pentamethyl cyclopentadienyl ring, MeOH represents methanol, MeI represent methyl iodide, MeOAc represents methyl acetate, Ac₂O represents acetic anhydride and AcOH represents acetic acid. measurements.

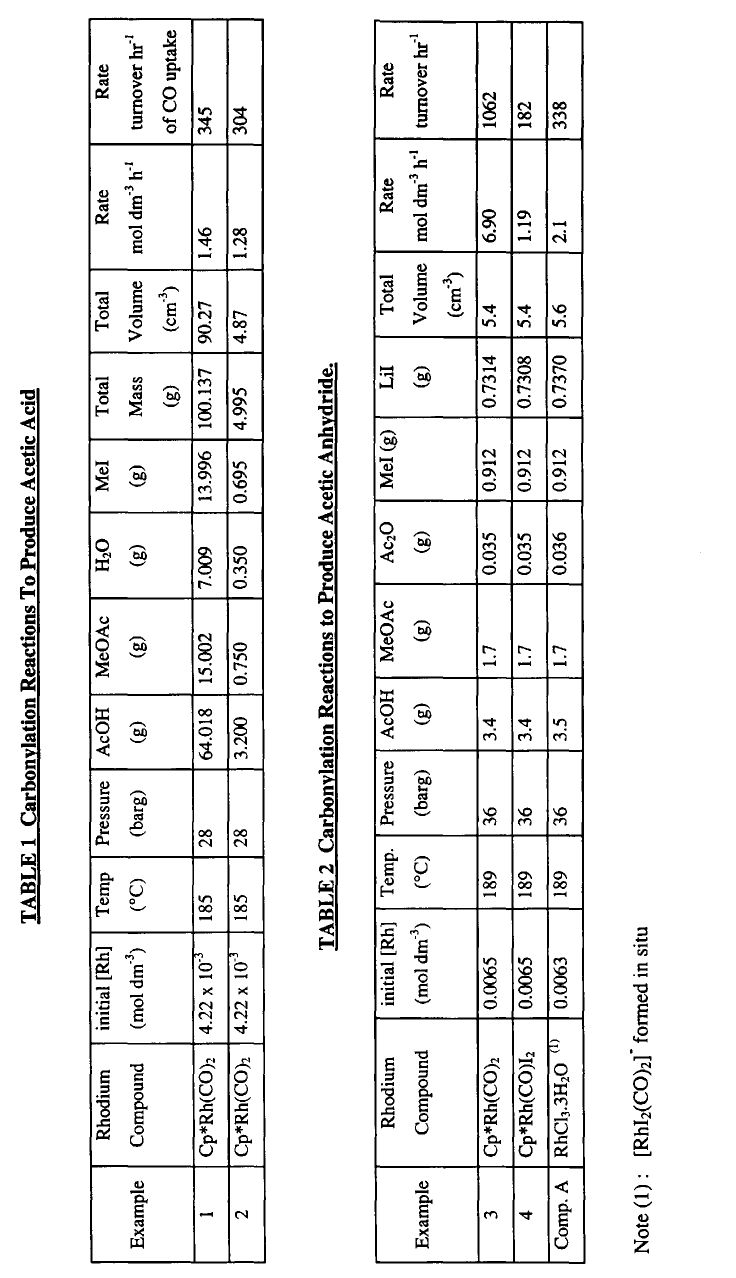

## Claims

1. A process for the carbonylation of an alcohol and/or reactive derivative thereof which process comprises contacting a liquid reaction composition comprising an alcohol and/or a reactive derivative thereof, a hydrocarbyl halide co-catalyst and optionally water, optionally with hydrogen at a molar ratio of hydrogen : carbon monoxide of less than 1 : 10, in the presence of a rhodium catalyst having the following structural formula (I) : 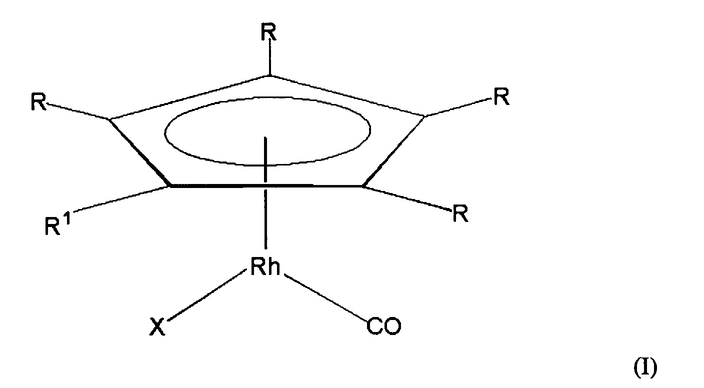 wherein :
the R groups are independently hydrogen, alkyl, aryl, substituted alkyl or aryl group; the R groups may be the same or different to each other;
R¹ is hydrogen, alkyl, aryl, substituted alkyl or aryl or a bridging group to X; and
X is a two electron donor which may optionally be linked to the cyclopentadienyl group via R¹.

2. A process as claimed in claim 1 in which in formula (I) R is alkyl, preferably methyl.

3. A process as claimed in claim 1 in which the cyclopentadienyl group is part of a larger ring system, preferably indenyl or flourenyl.

4. A process as claimed in any one of the preceding claims in which in formula (I) R¹ is methyl.

5. A process as claimed in any one of claims 1 to 3 in which in formula (I) the R¹ group is a bridging group to X.

6. A process as claimed in claim 5 in which R¹ has the formula (CR ²₂)ₙYₘ, wherein the R² groups are independently hydrogen, alkyl, aryl or substituted alkyl or aryl, and may be the same or different, n is an integer 0 to 5, Y is a group containing silicon, oxygen, nitrogen or sulphur and m is an integer 0 to 5.

7. A process as claimed in claim 5 or claim 6 in which in formula (I) X is PR''₂, NR''₂, SR'' or OR'' where R'' is alkyl, aryl or substituted alkyl or aryl which may be the same or different.

8. A process as claimed in any one of claims 1 to 4 in which in formula (I) X is CO, PR³₃, NR³₃, SR³₂ or OR³₂ where each R³ group is independently hydrogen, alkyl, aryl or substituted alkyl or aryl and the R³ groups which may be the same or different.

9. A process as claimed in any one of claims 7 and 8 in which in X the P, S, N and O atoms are part of a ring system.

10. A process as claimed in claim 9 in which the ring system is an aromatic system, preferably pyridine.

11. A process as claimed in any one of claims 1 to 4 in which X is a charged species, preferably a halide.

12. A process as claimed in any one of claims 1 to 11 modified in that the Rh of formula (I) is additionally bound to a Z group, such that Z = X = halide, preferably iodide.

13. A process as claimed in any one of claims 1 to 11 modified in that the Rh of formula (I) is additionally bound to a Z group, such that Z is halide, preferably iodide and in place of the CO group of formula (I) there is an acyl group of formula R⁴CO in which R⁴ is hydrogen, alkyl, aryl or substituted alkyl or aryl.

14. A process as claimed in claim 13 in which R⁴ is alkyl, preferably methyl.

15. A process as claimed in claim 1 in which the rhodium catalyst is Cp*Rh(CO)₂ or Cp*Rh(CO)I₂ in which Cp* is pentamethyl cyclopentadienyl group or in which in formula (I) R=R¹=H and X = CO.

16. A process as claimed in any one of the preceding claims in which the partial pressure of hydrogen, if present, is less than 2 bar.

17. A process as claimed in any one of the preceding claims in which the alcohol is methanol and there is produced acetic acid and/or methyl acetate.

18. A process as claimed in any one of claims 1 to 16 in which methyl acetate and/or dimethyl ether is used optionally with methanol and/or water and there is produced acetic anhydride and optionally acetic acid.
